# EUROPEAN PATENT APPLICATION

(11) **EP 4 678 963 A1**
(43) Date of publication of application: **14.01.2026**
(21) Application number: 25210768.5
(22) Date of filing: 07.02.2023
(51) Int. Cl.: F16L 25/00, F16L 11/118, A61M 16/08, A61M 39/08, B29C 53/58, F16L 11/16, F16L 57/02, F16L 33/32, B29C 53/78, A61M 16/10, A61M 39/10

(54) **FLEXIBLE HOSE WITH EMBEDDED HELICAL CONDUCTORS AND METHOD OF MANUFACTURING SAME**

(30) Priority: 09.02.2022 EP 22156000
(62) Divisional of application: 23703580.3
(71) Applicant: Plastiflex Group, 3583 Paal-Beringen (BE)
(72) Inventor: SONG, Chunfang, 2600 Berchem (BE); VANGERVEN, Jan, 3520 Zonhoven (BE); RIEFFE, Milan, 3970 Leopoldsburg (BE); DIELS, Domin, 2275 Gierle (BE)
(74) Representative: Sarlet, Stephanie

(57) **Abstract**

A flexible plastic hose comprising a flexible hose wall comprising a thin-walled tubular body with a helical outer support rib and a cuff connected to a first extremity of the tubular body. The cuff comprises a connector portion through which the tubular body is connectable to an external device and a strain relief portion located at an entrance side of the cuff where the tubular body enters the cuff, wherein the strain relief portion has, at least partially, an internal screw-thread complementary to the helical reinforcing rib and provided for holding a portion of the first extremity of the tubular body without being bonded thereto. The strain relief portion comprises at least one shell which is integrally formed with the connector portion and is hingedly connected thereto and which covers at least part of the tubular body in circumferential direction.

## Description

### Technical field

The present invention generally relates to collapse-resistant, flexible thermoplastic hoses which include a plurality of conductors extending helically along the length of the hose, and to methods for manufacturing such flexible hoses.

### Background art

Hoses having a relatively thin wall and an integral helical supporting rib are known in the art. The thin wall generally provides flexibility while the supporting rib generally provides substantial crush resistance, while leaving the wall flexible enough to permit short-radius bends without collapsing or kinking the hose. In medical applications, it is desirable that the inside of the hose is free of crevices in which soil or bacterial contamination could reside, so that a clean environment can be obtained. A smooth inner bore for the hose is also important because flow resistance is preferably kept to a minimum when a tidal air flow is conducted through the hose, as is the case with inhalation therapy and other breathing-assistance machines.

It is further known to integrate conductors into the rib of the hose, for example from the following documents.

US5454061A discloses a helically wound and helically ribbed plastic tubing incorporating an electrically conductive heating wire and an apparatus and method for making the tubing. A plastic ribbon is wound about an axis into a tube with one edge of each lap overlapping and heat-bonded to an edge of the preceding lap as the tubing is rotated; a conductive wire is embedded in the ribbon; and a bead is applied and heat-sealed onto the tubing, encapsulating the conductive wire during rotation of the tubing, providing a unitary structure including a conductive wire integral to a flexible tubing having a corrugated crevice-free outside and a smooth inside. Coolant is applied to the tubing for cooling the unitary ribbon, wire and bead tubing and to assist in advancing the tubing along the axis.

US7965930B2 discloses a plastic tubing with helical support bead which incorporates plural electrical conductors outside of the tubing bore and insulated from ambient, and from one another. The conductors may have a desired relatively high thermal conductivity to tidal air flow within the tubing, while also having a comparatively high thermal resistance to ambient. A method for making the tubing includes extruding a molten thermoplastic ribbon with an elevated plateau portion defining plural conductor-receiving grooves. The plastic ribbon is wrapped to form a tube and plural conductors are embedded in the grooves of the plateau portion. A molten thermoplastic bead is then applied atop the plateau portion.

EP1741462A1 discloses a flexible duct with devices for heating the conveyed stream and provided with elements for transmitting the parameters sensed by means of sensing probes connected thereto. The duct comprises a flexible tubular body, on the outer surface of which a wirelike stiffening element and at least one heating filament are distributed substantially in a helical arrangement. The duct comprises at least one cable, which is distributed on the outer surface of the tubular body substantially along a helix and has at least a first end connected to at least one respective probe and a second end connected to a control and management unit.

It has been found that in prior art hoses the positioning of the conductors or wires in the end product may vary. It is believed that this variance may be caused by residual heat in the materials used for manufacturing the hoses, so that the conductors or wires can "travel" in the still at least partially molten material.

Further, it has been found that the smoothness of the inner bore of the prior art hoses may be insufficient.

Further, during manufacturing of such flexible hoses, the steps for connecting the conductors or wires which are included in the helical support rib to an interface or cable on one of the connectors or cuffs of the hose may be labour intensive.

### Disclosure of the invention

It may be aim of the present invention to provide flexible plastic hoses which do not show at least one of the drawbacks of prior art hoses.

It may be another aim of the present invention to provide a method for manufacturing such flexible plastic hoses.

It may be another aim of the present invention to provide a simpler method for applying a connector or cuff to an end of the hose body.

It may be another aim of the present invention to provide a simpler method for connecting the conductors or wires of such hose to an interface or cable provided on a connector or cuff of the hose.

These and other aims may be achieved with the subject-matter of the independent claims. Preferred embodiments are defined in the dependent claims.

The present disclosure provides, in an aspect, which may be combined with other aspects and embodiments described herein, a flexible plastic hose comprising a flexible hose wall formed of a helically wound ribbon and a helical outer support rib, and a plurality of conductors or conductive wires helically disposed within said support rib, wherein adjacent windings of the ribbon are attached to each other by means of a weld material such that an elongate tubular body is formed, preferably having a substantially smooth bore, wherein the support rib is positioned on top of the weld material and comprises a lower rib portion, comprising a plurality of grooves wherein the conductors are disposed, and an upper rib portion on top of the lower rib portion and the conductors, and wherein the weld material attaches the lower rib portion on the tubular body.

In embodiments according to the disclosure, the lower rib portion which has the grooves and the conductors disposed in the grooves, is a part which is, during manufacturing, formed and allowed to cool down to a certain extent before it is subjected to the helically winding step. Preferably, the manufacturing process is controlled such that the material of the lower rib portion has sufficiently solidified in order to prevent the "travel" of the conductors or wires. It has been found that such travel could be caused by residual heat present in the lower rib portion, which residual heat was required in prior art processes for attachment or welding purposes. In embodiments according to the disclosure, a separate welding material is used, i.e. a separate part from the lower rib portion, for attaching the lower rib portion on the tubular body, such that the lower rib portion can be allowed to cool down and that the travel of the conductors or wires can be prevented. In this way, a correct positioning of the conductors or wires in the rib can be ensured, which may have advantages in manufacturing, namely facilitating automation of steps for connecting the conductors or wires to an interface or the like, as well as in the finished hose, as the risk of an exposed conductor or wire on the inside of the hose can be minimised.

Furthermore, in embodiments according to the disclosure, it has been found that by the use of the welding material for attaching the adjacent windings or turns of the ribbon to each other and/or attaching the lower rib portion on the tubular body formed by the adjacent windings of the ribbon, smoothness of the bore of the hose may be improved, because the welding material which is applied in molten state can flow and smoothen ridges or crevices on the inside of the tubular body.

In embodiments of the disclosure, the weld material may form a lower layer of the support rib, underneath the lower rib portion.

In embodiments, the lower rib portion may be a separate part from the helically wound ribbon, attached thereto by means of the weld material.

In embodiments, the adjacent windings of the ribbon have opposite side edges which are spaced from each other in axial direction of the hose and are attached to each other by means of the weld material. This means that in such embodiments the adjacent windings of the ribbon are not overlapping each other.

In embodiments, the adjacent windings of the ribbon have opposite side edges which overlap each other in axial direction of the hose and are attached to each other by means of the weld material.

In embodiments, the lower rib portion of the helical support rib may be an integral part of the helically wound ribbon. The lower rib portion may be provided at one of the side edges of the ribbon.

In embodiments, the ribbon and the lower rib portion are made of a first thermoplastic material. This means that the ribbon and the lower rib portion may be made of the same thermoplastic material. Preferred thermoplastics are disclosed elsewhere herein.

In embodiments, the weld and the upper rib portion are made of a second thermoplastic material. This means that the weld and the upper rib portion may be made of the same thermoplastic material, which is a second thermoplastic material, preferably different from the first thermoplastic material. Preferred thermoplastics are disclosed elsewhere herein.

The present disclosure provides, in another aspect, which may be combined with other aspects and embodiments described herein, a flexible plastic hose comprising a flexible hose wall which comprises an elongate tubular body, preferably having a substantially smooth bore, formed of a helically wound ribbon of which adjacent windings, which may or may not overlap each other, are attached to each other by means of a weld material. The hose further comprises a helical outer support rib with at least one conductor or conductive wire helically disposed within said support rib. The support rib is formed of a lower rib portion and an upper rib portion positioned on top of the lower rib portion. The lower rib portion may comprise one or more grooves wherein the at least one conductor is/are disposed, preferably one conductor or conductive wire in each groove. The lower rib portion may be positioned on top of a region of the tubular body where the adjacent windings of the ribbon are attached to each other and may be attached to the body by means of the weld material, or the lower rib portion may be an integral part of the ribbon, preferably at one side edge of the ribbon.

The present disclosure provides, in another aspect, which may be combined with other aspects and embodiments described herein, a method of manufacturing a flexible plastic hose comprising a flexible hose wall formed of a helically wound ribbon and a helical outer support rib, and a plurality of conductors helically disposed within said support rib, the method comprising: helically winding the ribbon; attaching adjacent windings of the ribbon by means of a weld material such that an elongate tubular body is formed having a substantially smooth bore; positioning a lower rib portion of a support rib on top of the weld material, wherein the lower rib portion comprises a plurality of grooves; disposing the conductors in the grooves; and covering the lower rib portion and the conductors by means of an upper rib portion; wherein the weld material attaches the lower rib portion on the tubular body.

In embodiments according to the disclosure, the adjacent windings of the ribbon may not be overlapped with each other, wherein the weld material attaches the adjacent windings of the ribbon and the lower rib portion to each other.

In embodiments according to the disclosure, the lower rib portion may be an integral part of the helically wound ribbon, wherein the adjacent windings of the ribbon are overlapped and attached to each other by means of the weld material.

The present disclosure provides, in another aspect, which may be combined with other aspects and embodiments described herein, a flexible plastic hose comprising a flexible hose wall comprising a thin-walled tubular body with a helical outer support rib and a plurality of conductors helically disposed within said support rib, and a cuff connected to a first extremity of the tubular body and comprising a cable interface portion provided for connecting a cable to the cuff and a platform portion where the conductors are connected to connector pads of the cable interface, wherein over a first portion at the first extremity of the tubular body the helical outer support rib is flattened with respect to a second, subsequent portion of the tubular body.

It has been found that by flattening the helical outer support rib over a first portion of the tubular body where the cuff is provided, the need for a strain relief element can be avoided.

In embodiments, the height of the helical outer support rib over the first portion of the tubular body is reduced to such an extent that the adjacent flattened ribs touch each other. For example, height of the helical outer support rib over the first portion of the tubular body may be reduced by at least 20%, 30%, 40%, preferably at least 50% of the height of the helical outer support rib over the second portion of the tubular body. The rib of the tubular body (the second portion) has a certain dimension to give the body a desired hub strength (or crush resistance, i.e. to keep the lumen of the body open during use). Depending on the dimensions of the rib, when adjacent ribs are flattened and touching each other this will result in a certain wall thickness of the flattened part (the first portion).

In embodiments, the first portion of the tubular body may comprise at least one turn, preferably at least two turns of the helical outer support rib. The length of the flattened portion depends on the circumstances, e.g. on the desired length of the cuff or portion that is overmoulded in a subsequest step (to cover the wire platform).

In embodiments, the first portion of the tubular body may extend beyond the cuff by at least one half turn, preferably at least one turn of the helical out support rib. Alternatively, this may be defined as that at least one half turn, preferably at least one turn of the flattened support rib is not covered by the cuff. Alternatively, this may be defined as that the first portion of the tubular body comprises x (flattened) turns of the helical outer support rib, whereas at most x-1/2 turns, preferably at most x-1 turns of the flattened helical outer support rib are located inside the cuff.

The present disclosure provides, in another aspect, which may be combined with other aspects and embodiments described herein, a flexible plastic hose comprising a flexible hose wall comprising a thin-walled tubular body with a helical outer support rib and preferably a plurality of conductors helically disposed within said support rib, a cuff connected to a first extremity of the tubular body and comprising: a connector portion through which the hose is connectable to an external device and a strain relief portion located at an entrance side of the cuff where the tubular body enters the cuff, the strain relief portion having, at least partially, an internal screw-thread complementary to the helical reinforcing rib and provided for holding a portion of the first extremity of the tubular body without being bonded thereto, wherein the strain relief portion comprises at least one shell which is integrally formed with the connector portion and hingedly connected thereto and which covers at least part of the tubular body in circumferential direction.

In embodiments, the strain relief portion may comprise two or more of such shells which are hingedly connected to the connector portion, preferably wherein the shells together cover substantially the whole tubular body in circumferential direction.

In embodiments, the shells may be fixed to each other by means of for example a snap connector.

In embodiments, the strain relief portion may comprise at least one of such shell(s) which is/are integrally formed with the connector portion and hingedly connected thereto, and at least one separate portion fixed to the shell(s) by means of for example a snap connector, preferably wherein the shell(s) and the separate portion together cover substantially the whole tubular body in circumferential direction.

In embodiments, the strain relief portion may comprise a first shell which is hingedly connected to the connector portion and at least one second shell which is/are hingedly connected to the first shell, preferably wherein at least two of the first and second shells are fixed to each other by means of for example a snap connector, preferably wherein the first and second shells together cover substantially the whole tubular body in circumferential direction.

In embodiments, the cuff may comprise an overmoulded portion covering at least part of the connector portion and/or at least part of the strain relief portion.

In embodiments, the overmoulded portion may comprise an inner portion which extens through one or more openings in the connector portion and/or the strain relief portion and is bonded to the tubular body.

The present disclosure provides, in another aspect, which may be combined with other aspects and embodiments described herein, a flexible plastic hose comprising a flexible hose wall comprising a thin-walled tubular body with a helical outer support rib and a plurality of conductors helically disposed within said support rib, and a cuff connected to a first extremity of the tubular body and comprising: a cable interface portion provided for connecting a cable to the cuff and a platform portion where the conductors are connected to connector pads of the cable interface, wherein the platform portion comprises a rib section with a series of ribs for guiding the individual conductors and separating the conductors from each other, wherein each rib comprises a hook portion at a distal end for holding one of the conductors, and wherein each connector pad of the cable interface portion comprises a slot for engaging one of the conductors, and wherein each conductor extends along one of the ribs, behind the respective hook portion and into a respective one of the slots.

### Brief description of the drawings

The invention will be further elucidated by means of the following description and the appended figures.
Figure 1 shows a first embodiment of a flexible plastic hose according to the present disclosure.
Figure 2 shows a cross-sectional view of the hose of Fig. 1.
Figures 3-5 show details of the view of Fig. 2.
Figure 6 shows an exploded cross-sectional view of the hose of Fig. 1.
Figure 7 shows a second embodiment of a flexible plastic hose according to the present disclosure.
Figure 8 shows a cross-sectional view of the hose of Fig. 7.
Figures 9-11 show details of the view of Fig. 8.
Figure 12 shows an exploded cross-sectional view of the hose of Fig. 7.
Figure 13 shows an exploded cross-sectional view of a further embodiment of a flexible plastic hose according to the present disclosure.
Figs. 14-16 schematically show embodiments of a flexible hose according to the present disclosure, comprising a strain relief at the cuff, and some steps of a method of manufacturing such a flexible hose.
Figs. 17-24 show, by means of subsequent perspective views, another embodiment of manufacturing a strain relief at the cuff of a flexible hose according to the present disclosure. These figures further show another aspect according to the present disclosure relating to steps for connecting the conductors of the tubular body of a flexible hose to a cable interface on the hose cuff.
Figs. 25-28 show schematically some alternative embodiments, in cross-section, of the embodiment of Figs. 17-24.

### Modes for carrying out the invention

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn on scale for illustrative purposes. The dimensions and the relative dimensions do not necessarily correspond to actual reductions to practice of the invention.

Furthermore, the terms first, second, third and the like in the description and in the claims, are used for distinguishing between similar elements and not necessarily for describing a sequential or chronological order. The terms are interchangeable under appropriate circumstances and the embodiments of the invention can operate in other sequences than described or illustrated herein.

Moreover, the terms top, bottom, over, under and the like in the description and the claims are used for descriptive purposes and not necessarily for describing relative positions. The terms so used are interchangeable under appropriate circumstances and the embodiments of the invention described herein can operate in other orientations than described or illustrated herein.

Furthermore, the various embodiments, although referred to as "preferred" are to be construed as exemplary manners in which the invention may be implemented rather than as limiting the scope of the invention.

The term "comprising", used in the claims, should not be interpreted as being restricted to the elements or steps listed thereafter; it does not exclude other elements or steps. It needs to be interpreted as specifying the presence of the stated features, integers, steps or components as referred to, but does not preclude the presence or addition of one or more other features, integers, steps or components, or groups thereof. Thus, the scope of the expression "a device comprising A and B" should not be limited to devices consisting only of components A and B, rather with respect to the present invention, the only enumerated components of the device are A and B, and further the claim should be interpreted as including equivalents of those components.

### General overview

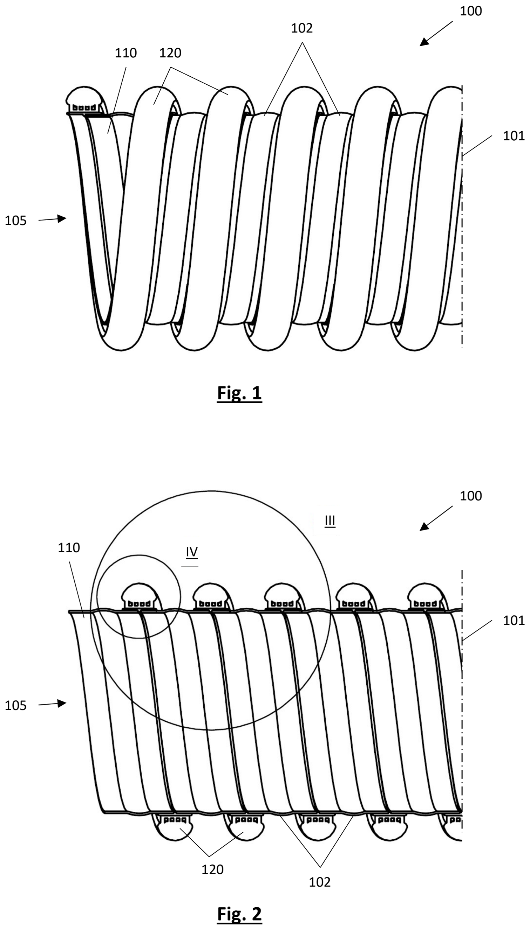

A first embodiment according to the present disclosure will be described with reference to Figs. 1-6.

Fig. 1 shows a portion of a flexible plastic hose 100 comprising a flexible hose wall 105, which is a body 101 formed of a helically wound ribbon 110 and a helical outer support rib 120, and a plurality of conductors 130 or conductive wires helically disposed within the support rib 120. The support rib provides the hose with a certain crush resistance, which is the resistance a hose provides against collapsing of the hose under lateral pressure, such as when one would stand on a hose laying on the floor, or against vacuum on the inside of the bore. Crush resistance may also be called hub strength or hoop strength, and is an important feature for a flexible hose in many of its applications. Embodiments according to the disclosure may comprise one or more helical support ribs, at least one of which has at least one conductor. In between the support ribs, or adjacent turns thereof, the wall of the hose comprises thin-walled web sections, which are formed by portions of one or more helically wound ribbons. The at least one helical support rib 120 has predetermined properties (e.g. thickness, material, pitch, etc) for providing sufficient mechanical strength and stiffness to the hose body 101. The thin-walled web sections 102 in between the rib windings provide the hose with the desired flexibility. As shown in the drawings, the web sections 102 may slightly bulge or comprise a fold in the middle, in order to increase flexibility.

Adjacent windings of the ribbon 110 are attached to each other by means of a weld material 103 such that an elongate tubular body 101 is formed, preferably having a substantially smooth bore. The support rib 120 is positioned on top of the weld material 103 and comprises a lower rib portion 121, comprising a plurality of grooves 123 wherein the conductors 130 are disposed, and an upper rib portion 122 on top of the lower rib portion 121 and the conductors 130. The weld material 103 also attaches the lower rib portion 121 on the tubular body 101.

In the embodiment of Figs. 1-6, the weld material 103 forms a lower layer of the support rib 102, underneath the lower rib portion 121 which is a separate part from the helically wound ribbon 110, attached thereto by means of the weld material 103. The adjacent windings of the ribbon 110 have opposite side edges which are spaced from each other in axial direction of the hose by a gap 104 of a given distance D, i.e. the adjacent windings of the ribbon 110 are not overlapping each other but are attached to each other by means of the weld material 103. The non-overlapping of the adjacent windings is preferred in view of a smoother inner bore, because otherwise a ridge would be formed at the overlap. The distance D of the gap 104 is chosen so as to ensure that the adjacent windings of the ribbon 110 do not become overlapped during manufacturing. The distance D is preferably chosen in a range of 50% to 200%, preferably 75% to 150% of the thickness of the ribbon at the side edges.

As shown in the Figures, the weld material 103 at least partly fills the gap 104 between the adjacent windings of the ribbon 110, thus reducing the recess or crevice which would otherwise be present, and making the inner wall of the hose more smooth. Preferably, the weld material 103 substantially completely fills the gap 104.

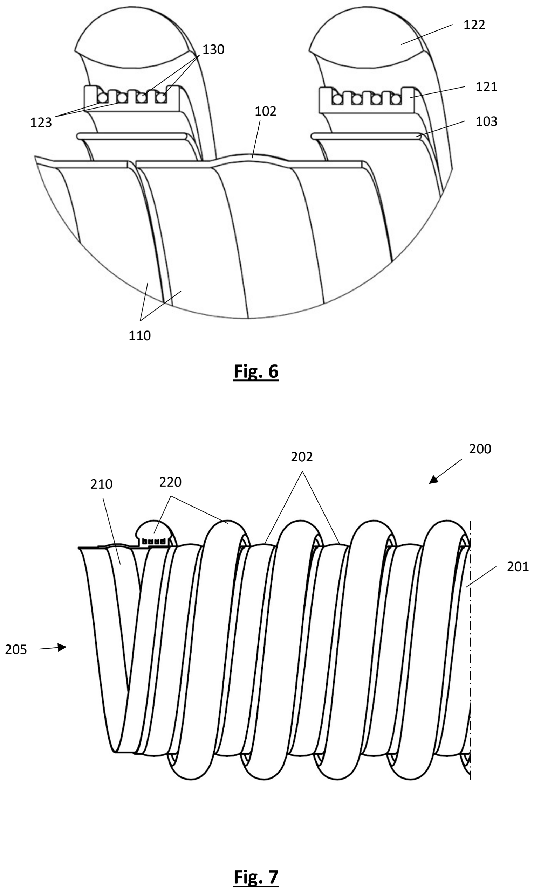

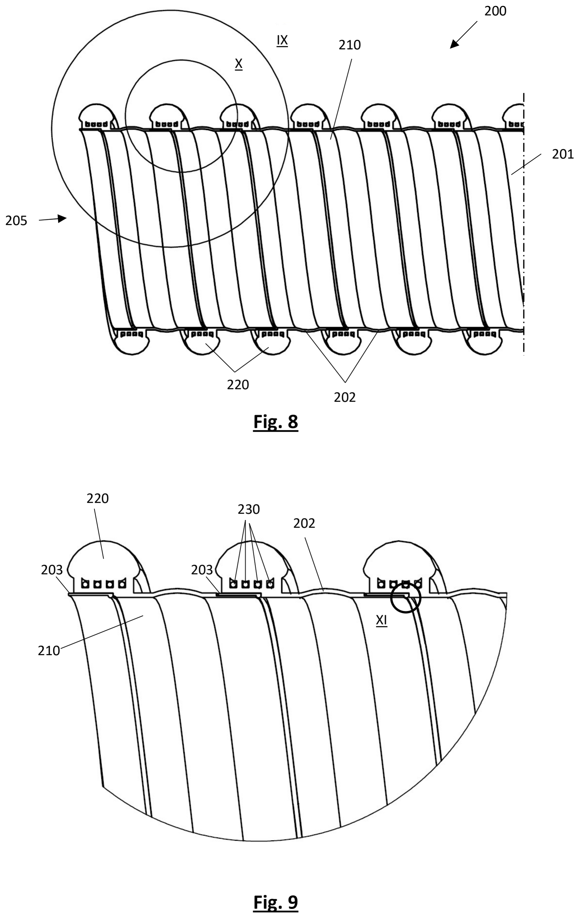

A second embodiment according to the present disclosure will be described with reference to Figs. 7-12, wherein the adjacent windings of the ribbon have opposite side edges which do overlap each other in axial direction.

Fig. 7 shows a portion of a flexible plastic hose 200 comprising a flexible hose wall 205, which is a body 201 formed of a helically wound ribbon 210 and a helical outer support rib 220, and a plurality of conductors 230 or conductive wires helically disposed within the support rib 220. The support rib provides the hose with a certain crush resistance. Embodiments according to the disclosure may comprise one or more helical support ribs, at least one of which has at least one conductor. In between the support ribs, or adjacent turns thereof, the wall of the hose comprises thin-walled web sections 202, which are formed by one or more helically wound ribbons 210. The at least one helical support rib 220 has predetermined properties (e.g. thickness, material, pitch, etc) for providing sufficient mechanical strength and stiffness to the hose body 201. The thin-walled web sections 202 in between the rib windings provide the hose with the desired flexibility.

Adjacent windings of the ribbon 210 are attached to each other by means of a weld material 203 such that an elongate tubular body 201 is formed, preferably having a substantially smooth bore. The support rib 220 is positioned on top of the weld material 203 and comprises a lower rib portion 221, comprising a plurality of grooves 223 wherein the conductors 230 are disposed, and an upper rib portion 222 on top of the lower rib portion 221 and the conductors 230. The weld material 203 also attaches the lower rib portion 221 on the tubular body 201.

In this embodiment, the lower rib portion 221 of the helical support rib is an integral part of the helically wound ribbon 210, in particular at one side edge of the ribbon. This side edge is overlapped with the opposite side edge of the preceding winding of the ribbon, and the overlapping portions are attached to each other by means of the weld material 203.

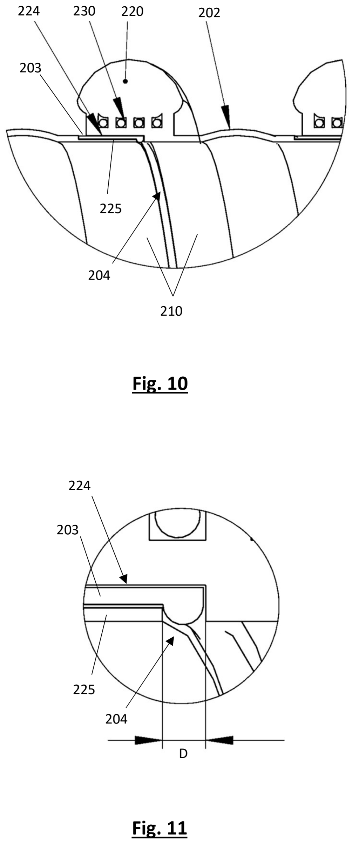

More in particular, in order to obtain a substantially smooth bore, a recess 224 may be provided in the bottom side of the ribbon 210, underneath at least a portion of the lower rib portion 221, to accommodate the opposite side edge 225 of the preceding winding of the ribbon 210 and the weld material 203 which attaches the two windings to each other. The recess 224 extends axially from the side edge of the ribbon and may for example span 20 to 80%, preferably 30 to 70%, more preferably at least 50% of the width of the lower rib portion 221. The opposite side edge 225 of the ribbon 210 may be thinned with respect to the general thickness of the ribbon 210, measured at the web section 202, for example over substantially the same length as the length of the recess 224 (in axial direction of the hose). As shown in Figs. 10 and 11, a gap 204 may still be present between the side edge 225 and the edge of the recess 224. The distance D of the gap 204 is chosen so as to ensure that the side edge 225 does not become overlapped with the edge of the recess 224 during manufacturing. The distance D is preferably chosen in a range of 50% to 200%, preferably 75% to 150% of the thickness of the ribbon at the web section 202.

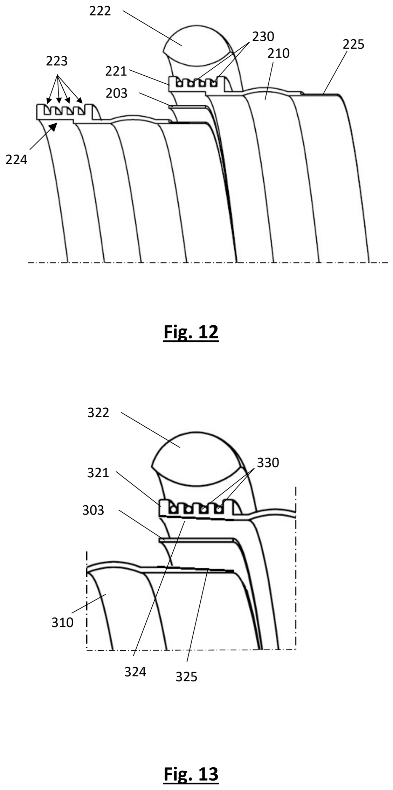

In a further embodiment, shown in Fig. 13, wherein the lower rib portion 321 is likewise and integral part of the ribbon 310, the bottom side of the lower rib portion 321 may be slanted and the opposite side edge portion 325 of the ribbon 310 may be correspondingly tapered, such that the after welding the two portions of the ribbon 310 onto each other a substantially smooth inner surface is achieved. In this embodiment, the side edge portion 325 of the ribbon 310 may substantially fully overlap with the lower rib portion 321.

In the embodiments shown, the lower rib portions 121, 221, 321 comprise four grooves 123, 223, 323, each including one conductor 130. In embodiments, the lower rib portions may comprise one, two, three, four, five or more grooves. Conductors or wires may be provided in each of the grooves, preferably one per groove, but some of the grooves may also be left empty. The conductors or wires 130, 230, 330 may be used for heating, conducting signals, conducting power, sensing (e.g. PTC or NTC wires), or other purposes. The upper rib portion 122, 222, 322 covers the grooves and conductors in order to insulate them from the environment.

The hoses according to the present disclosure may comprise cuffs (not shown) at both ends of the hose for coupling the hoses with further devices or equipment, depending on the intended application. At least one of the cuffs may comprise an interface via which the at least one conductors or conductive wires, which are integrated into the at least one helically wound rib, are connectable to further devices or equipment. At one or both ends, the cuff may furthermore be provided with a strain relief element.

In embodiments, the hoses according to the present disclosure may be suitable for medical applications such as CPAP or respiratory care, to which end the cuffs are then made suitable for connecting to for example CPAP or respiratory devices, further tubing and/or patient interfaces.

Suitable cuffs and strain relief elements are for example known from WO 2012/143563 A1, by the same applicant, which is incorporated herein by reference in its entirety.

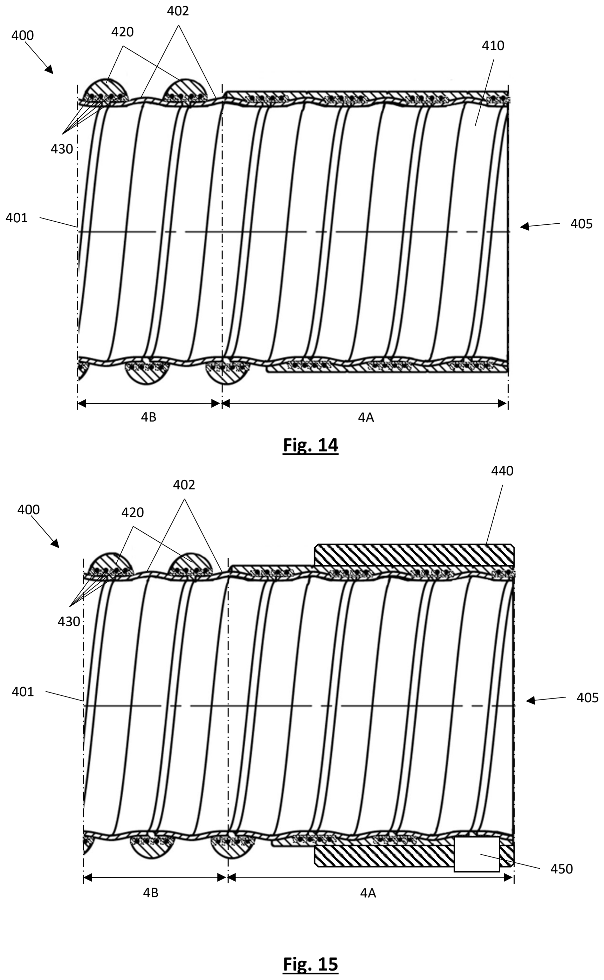

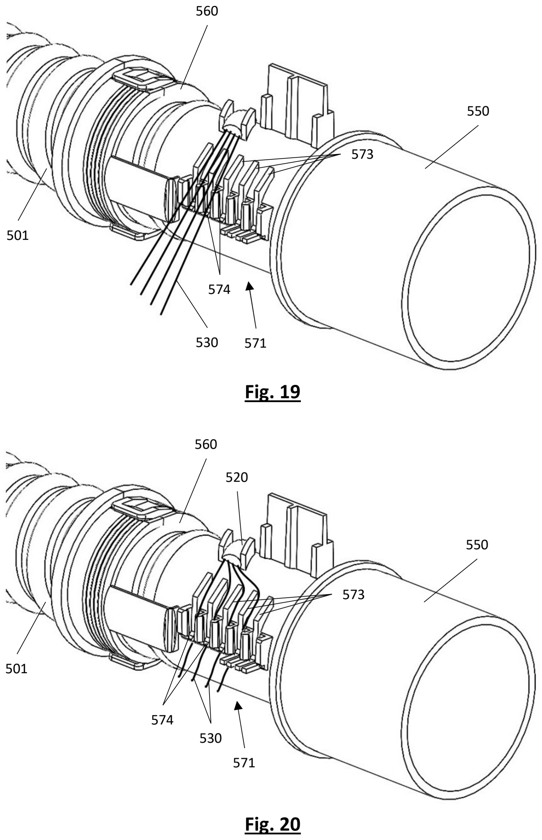

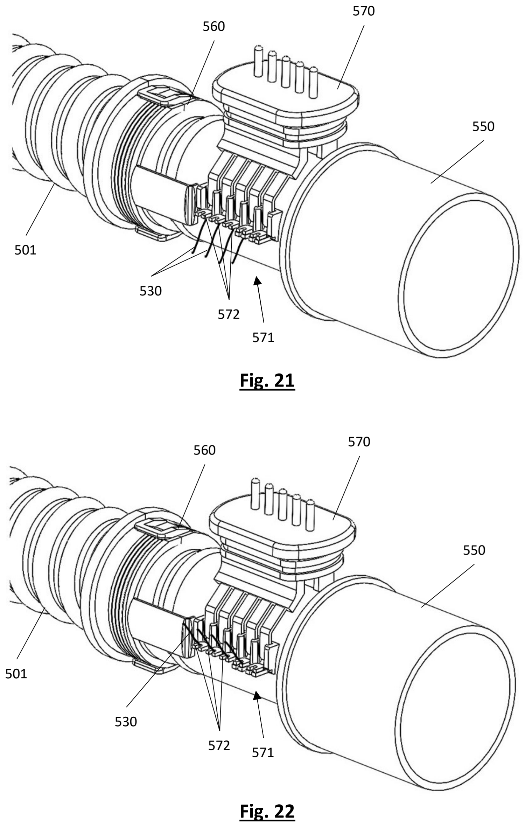

Other suitable cuffs and strain relief solutions are described below with reference to figures 14-28.

### Materials

In embodiments according to the present disclosure, the ribbon 110, 210 310 and the lower rib portion 121, 221, 321 may be made of the same, first thermoplastic material. This is in particular the case in embodiments where the lower rib portion is an integral part of the ribbon.

In other embodiments, the ribbon and the lower rib portion may also be made of different thermoplastic materials.

In embodiments according to the present disclosure, the weld 103, 203, 303 and the upper rib portion 122, 222, 322 may be made of the same, second thermoplastic material. This second thermoplastic material may be different from the first thermoplastic material.

In other embodiments, the weld and the upper rib portion may also be made of different thermoplastic materials.

The different components, i.e. the ribbon, the lower rib portion, the upper rib portion and the weld material may comprise at least one thermoplastic material, or a blend of thermoplastic materials, for example selected from the group consisting of: polyethylene (PE), water vapour permeable PE (trade name Arnitel), thermoplastic polyester, polypropylene (PP), polyamide, polyvinylchloride (PVC), preferably flexible PVC, or other thermoplastic materials known by persons skilled in the art, preferably any thermoplastic material approved according to ISO 10993 for use in medical applications. Suitable materials are mentioned in WO 2020/094741 A1, by the same applicant, which is incorporated herein by reference in its entirety.

In particular, the ribbon 110, 210, 310 and/or the lower rib portion 121, 221, 321 may be made of a first polymer material or blend selected for making the ribbon and/or lower rib portion permeable to water vapor and possibly one or more further breathing gases. In embodiments, the predetermined material or blend may be selected from the group of Kraton D2104, Kraton D1 101, Kraton G1652, Kraton G2705, Estane 58245, Estane MVT (such as MVT 90 NT1 , MVT 80 NT1 or MVT 75AT3) Pebax MV 3000 SP 01 , Arnitel VT (such as VT3108, VT31 18 or VT 7812), Pebax MV6100, Butadiene TPE, Nylon 66, Cyclolac 1033, Hytrel 5556, PET, PVDF, EAA, PP, FEP, LCP, TPU (e.g. polyether TPU) and PTFE, preferably Arnitel VT3108. In preferred embodiments, the predetermined material is selected from one or more of the following commercially available material brands: Hytrel, Arnitel. In more preferred embodiments the predetermined material is one or more of the following commercially available materials: Arnitel VT3108, Arnitel VT31 18, Arnitel VT7812 and Arnitel VT3104, preferably one or more of Arnitel VT3108 and Arnitel VT3104, most preferably Arnitel VT3108.

In embodiments, for example in embodiments where the ribbon is made of such a water vapour permeable material, the weld 103, 203, 303 and/or the lower rib portion 121, 221, 321 may be made of an electrically insulating polymer material. This may be advantageous to avoid short circuits in cases where the water vapour permeable material becomes humid due to absorption or permeation of the water vapour.

In embodiments, for example in embodiments where the ribbon and/or the lower rib portion are made of such a water vapour permeable material, the conductors or wires 130, 230, 330 may be covered in an electrically insulating coating. This may be advantageous to avoid short circuits in cases where the water vapour permeable material becomes humid due to absorption or permeation of the water vapour.

In embodiments, the materials of the components may be chosen to facilitate recycling of the product after use. For example, a degradable material may be used which breaks upon application of UV light, a certain wavelength of light, heat, a solvent or a chemical. Such a material could advantageously be used for the weld 103, 203, 303 and/or the lower rib portion 121, 221, 321 and/or the upper rib portion 122, 222, 322. The degradation of the material would break the weld or bond between the components of the sandwich design (weld, lower rib portion, upper rib portion) and facilitate the separation of the copper wires upon recycling.

In embodiments, the weld 103, 203, 303 and/or the upper rib portion 122, 222, 322 may be made of a high melt index material such as a high density polyethylene.

In embodiments, for example in embodiments where static electricity may occur on the inside of the hose, the material of the weld 103, 203, 303 may be made electrically conductive by adding conductive particles or fibres. For example, the weld may be made of a thermoplastic base material containing electrically conductive fibres or particles, such as for example carbon black or metallic particles, preferably 1 to 50% or 10 to 40% by weight of carbon black, more preferably 25 to 35% by weight of carbon black.

### Dimensions

In embodiments according to the present disclosure, the lumen of the hose may have a diameter of, for example 5 mm to 30 mm.

In embodiments according to the present disclosure, the rib 120, 220, 320 may e.g. have dimensions, i.e. width and/or height measured in cross section, in the range of 1.0-3.0 mm.

In embodiments according to the present disclosure, the ribbon portions which include the web sections 102, 202, 302, which have predetermined properties (e.g. dimensions, material, etc) chosen for providing desired flexibility to the hose body, may e.g. have the following dimensions. The width of the ribbon portions between two successive rib portions, measured in axial direction of the hose, may e.g. be in the range of 1.0-10.0 mm, preferably 3.0 to 8.0 mm, more preferably 4.0-6.0 mm.

In embodiments, the pitch p of the reinforcing ribs of the flexible plastic hoses according to the present disclosure, measured from peak to peak in axial direction of the hose, is preferably in the range of 3.0 to 9.0 mm, more preferably 4.0 to 8.0 mm, even more preferably 5.0 to 7.0 mm.

The radial thickness of the ribbon, in particular the web sections 102, 202, 302 is preferably in the range of 0.05 to 0.25 mm, more preferably 0.07 to 0.20 mm. This allows for a very flexible hose, which can easily bend over 90° under a weight of e.g. 0.100 kg or 0.200 kg. Optionally the web sections may be transparent, so that flow of the fluid (gas or liquid) inside the tube may be visible.

The grooves 123, 223, 323 in the lower rib portion may have dimensions in the range of 0.2 to 2.0 mm, for including conductors or wires with a diameter in the range of 0.1 to 1.5 mm.

### Manufacturing

The hoses shown in the Figures may be manufactured by means of methods of helically winding extruded parts, which are generally known in the art. For example, the parts shown in the exploded views of Figs. 6, 12 and 13 may be extruded and subsequently wound on a mandrel, in a way as described in US4162370A, US5454061A or US7965930B2.

Accordingly, a method of manufacturing a flexible plastic hose according to the present disclosure, for example as shown in the drawings, may comprise the steps of: helically winding the ribbon 110, 210, 310; attaching adjacent windings of the ribbon by means of a weld material 103, 203, 303 such that an elongate tubular body is formed having a substantially smooth bore; positioning a lower rib portion 121, 221, 321 of a support rib on top of the weld material, wherein the lower rib portion comprises one or more, preferably a plurality of grooves 123, 223, 323; disposing at least one conductor or conductive wire 130, 230, 330 in at least one the grooves; and covering the lower rib portion and the conductors by means of an upper rib portion 122, 222, 322; wherein the weld material 103, 203, 303 attaches the lower rib portion 121, 221, 321 on the tubular body 101, 201. The above mentioned steps are not necessarily carried out in the order in which they are mentioned. For example, in embodiments where the lower rib portion is an integral part of the ribbon, the step of positioning the lower rib portion on top of the weld material is part of the step of attaching the adjacent windings of the ribbon by means of the weld material.

In embodiments according to the disclosure, such as the embodiment shown in Figs. 1-6, the adjacent windings of the ribbon 110 may not be overlapped with each other, and the lower rib portion 121 may be extruded as a separate part, separate from the ribbon. In such embodiments, the same weld material 103 attaches the adjacent windings of the ribbon 110 and the lower rib portion 121 to each other. As described above, in such embodiments, the helical winding of the ribbon is preferably controlled such that a gap 104 is present between the adjacent windings, with a distance D.

In embodiments according to the disclosure, such as the embodiments shown in Figs. 7-13, the lower rib portion 221, 321 may be an integral part of the helically wound ribbon 210, 310. In such embodiments, the adjacent windings of the ribbon 210, 310 are overlapped and attached to each other by means of the weld material. In particular, the weld material 203, 303 is applied over the side edge portion 225, 325 of the ribbon and the subsequent winding of the ribbon is attached by positioning the lower rib portion 221, 321 over the side edge portion 225, 325 and the weld material 203, 303. As described above, the embodiment of Figs. 1-6, the helical winding of the ribbon 210 is preferably controlled such that a gap 204 is present between the side edge portion 225 and the opposing side edge of the recess 224 in the bottom side of the ribbon, at the lower rib portion 221.

In embodiments according to the disclosure, the ribbon 110, 210, 310 and the lower rib portion 121, 221, 321, irrespective of whether or not it is an integral part of the ribbon, are preferably allowed to cool down a certain amount before the helical winding step, at least such that the lower rib portion is not deformed by the application of the conductors or wires 130, 230, 330 in the grooves 123, 223, 323. Preferably, the ribbon and the lower rib portion are allowed to cool down to a temperature below the Vicat softening point of the material (the temperature at which the specimen is penetrated to a depth of 1 mm by a flat-ended needle with a 1 mm² circular or square cross-section, see ASTM D 1525 or ISO 306).

In other words, the lower rib portion 121, 221, 321 which has the grooves and the conductors disposed in the grooves, is a part which is, during manufacturing, formed, preferably by extrusion, and allowed to cool down to a certain extent before it is subjected to the helically winding step. Preferably, the manufacturing process is controlled such that the material of the lower rib portion has sufficiently solidified in order to prevent the "travel" of the conductors or wires. It has been found that such travel could be caused by residual heat present in the lower rib portion, which residual heat was required in prior art processes for attachment or welding purposes. In embodiments according to the disclosure, a separate welding material is used, i.e. a separate part from the lower rib portion, for attaching the lower rib portion on the tubular body, such that the lower rib portion can be allowed to cool down and that the travel of the conductors or wires can be prevented. In this way, a correct positioning of the conductors or wires in the rib can be ensured, which may have advantages in manufacturing, namely facilitating automation of steps for connecting the conductors or wires to an interface or the like, as well as in the finished hose, as the risk of an exposed conductor or wire on the inside of the hose can be minimised.

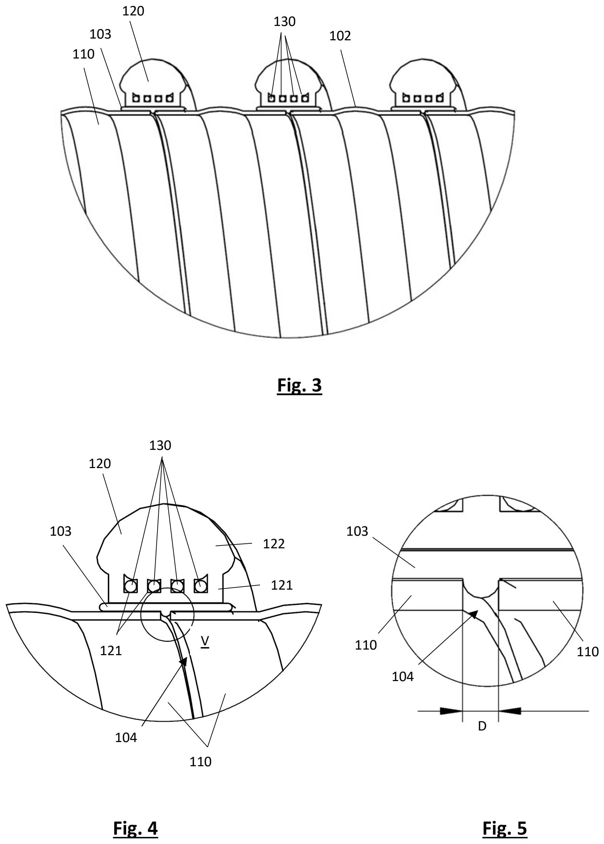

Furthermore, in embodiments according to the disclosure, it has been found that by the use of the weld material 103, 203, 303 for attaching the adjacent windings or turns of the ribbon to each other and/or attaching the lower rib portion on the tubular body formed by the adjacent windings of the ribbon, smoothness of the bore of the hose may be improved, because the welding material which is applied in molten state can flow and smoothen ridges or crevices on the inside of the tubular body. As described above, the helical winding of the ribbon is preferably controlled such that a gap 104, 204 is maintained between the adjacent windings or side edges, with a predetermined distance D, to avoid overlapping of the ribbon / side edges and obtain a smoother inside surface of the tubular body. The weld material applied in molten state can flow at least partially into this gap to substantially completely smoothen out the inside surface of the tubular body, as shown in Figs. 4 and 11.

### Strain Relief by flattening the rib

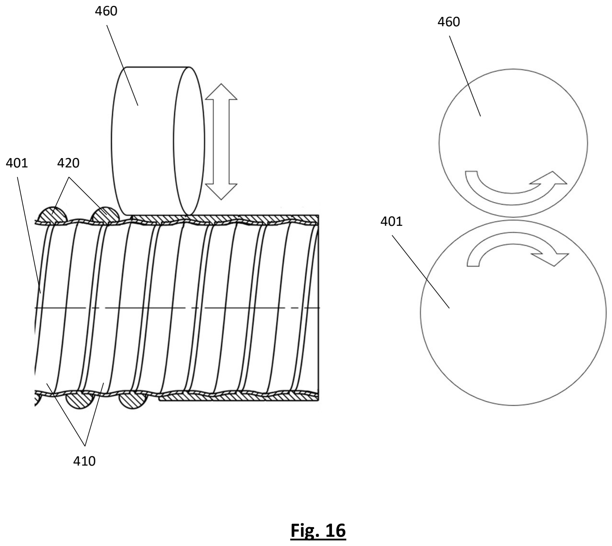

With reference to Figs. 14-16 a solution is described to provide a strain relief at the cuff of a flexible hose according to the present disclosure. The figures show a hose, and steps in the manufacturing thereof, which may be combined with other aspects and embodiments described herein. The flexible plastic hose 400 comprises a flexible hose wall comprising a thin-walled tubular body 401 with a helical outer support rib 420 and a plurality of wires or conductors 430 helically disposed within said support rib 420, and a cuff 440 connected to a first extremity of the tubular body and comprising a cable interface 450 provided for connecting a cable to the cuff. The cable interface 450 is only shown schematically and may for example comprise a platform portion where the conductors are connected to connector pads of the cable interface as described elsewhere herein. Over a first portion 4A at the first extremity 405 of the tubular body 401 the helical outer support rib 420 is flattened with respect to a second, subsequent portion 4B of the tubular body. The cuff 440 may be formed by overmoulding (part of) the second portion first portion 4A where the rib is flattened. Fig. 14 shows the hose extremity 405 after flattening according to Fig. 16; Fig. 15 shows the hose extremity 405 after manufacturing the cable interface 450 and overmoulding (part of) the flattened portion 4B to obtain the cuff 440. As shown, the tubular body 401 may be formed by a ribbon 410 and rib 420 construction as described elsewhere herein.

It has been found that by flattening the helical outer support rib over the first portion of the tubular body where the cuff is provided, a separate strain relief element, as described herein for other embodiments, may be avoided.

The flattening of the rib may be achieved, as shown in Fig. 16 in a side/cross-sectional view and in a frontal view, in a helical winding process in which a tubular body 401 is formed by helically winding a ribbon 410 and a rib 420, by applying a pressure on the outside of the rib while the rib material (at least the upper rib portion) is still in molten state, for example by pressing a roller element 460 on the outside of the rib 420. The roller element may be controlled to move back and forth (as shown by the arrow) repetitively each time a predetermined stretch of hose has been manufactured (corresponding to the desired length of the hoses), to obtain the flattened rib sections at the cuffs. In this way, the flattened rib sections 4A may be achieved by an in-line step of the hose manufacturing process. The applied pressure will result in a reduction of the height of the rib and an increase in the width of the rib, which may achieve the desired reinforcement of the web sections in between the rib and thus the desired strain relief.

In embodiments, the height of the helical outer support rib 420 over the first portion 4A of the tubular body is reduced to such an extent that the adjacent flattened ribs touch each other. For example, height of the helical outer support rib over the first portion 4A of the tubular body may be reduced by at least 20%, 30%, 40%, preferably at least 50% of the height of the helical outer support rib over the second portion 4B of the tubular body. The rib 420 of the tubular body (the second portion) has a certain dimension to give the body a desired hub strength (or crush resistance, i.e. to keep the lumen of the body open during use). Depending on the dimensions of the rib 420, when adjacent ribs are flattened and touching each other this will result in a certain wall thickness of the flattened part (the first portion).

In embodiments, the first portion 4A of the tubular body may comprise at least one turn, preferably at least two turns of the flattened helical outer support rib. The length of the flattened portion 4A depends on the circumstances, e.g. on the desired length of the cuff 440 or portion that is overmoulded in a subsequest step (to cover the wire platform).

In embodiments, the first portion 4A of the tubular body may extend beyond the cuff 440 by at least one half turn, preferably at least one turn of the helical out support rib.

### Strain relief element

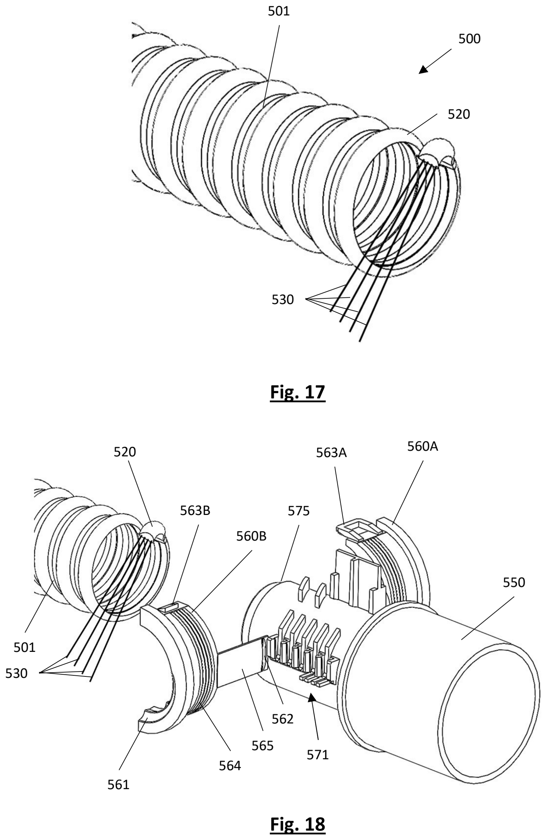

With reference to Figs. 17-28, another solution is described to provide a strain relief at the cuff of a flexible hose according to the present disclosure, in a way similar to the strain relief known from WO 2012/143563 A1 by the same applicant, the disclosure of which is incorporated herein by reference in its entirety. The figures show a hose 500, and steps in the manufacturing thereof, which may be combined with other aspects and embodiments described herein. The flexible plastic hose comprises a flexible hose wall comprising a thin-walled tubular body 501 with a helical outer support rib 520 and preferably at least one, more preferably a plurality of conductors 530 helically disposed within said support rib 520. The tubular body 501 may be manufactured according to other embodiments described herein. The hose comprises a cuff 540 connected to a first extremity of the tubular body 501 and comprising: a connector portion 550 through which the hose is connectable to an external device and a strain relief portion 560 located at an entrance side of the cuff where the tubular body 501 enters the cuff 540.

The strain relief portion 560 has, at least partially, an internal screw-thread 561 complementary to the helical reinforcing rib 520 and provided for holding a portion of the first extremity of the tubular body 501 without being bonded thereto. The internal screw-thread 561 may completely extend along one or more turns of the helical reinforcing rib 520. The internal screw-thread may also be an interrupted screw-thread formed by a plurality of protrusions or protruding screw-thread portions which protrude inwards from the inside wall of the strain relief portion, for holding one or more turns of the helical reinforcing rib.

In the embodiment of Fig. 17-24, the strain relief portion 560 comprises two shells 560A and 560B which are integrally formed with the connector portion 550 and hingedly connected thereto. In this way, the strain relief portion may be manufactured integrally with the connector portion in a single injection moulding step. The two shells 560A and 560B are bent along their respective hinges 562 and comprise a snap connection mechanism 563A-563B by which they are snap fitted to each other to form the strain relief portion 560. The two shells together cover the whole of the tubular body 501 in circumferential direction. Each shell 560A, 560B comprises a semi-cylindrical portion 564 at one end of a leg 565 with the hinge 562 at the other end of the leg. In this way, a portion of the hose extremity remains exposed for being fixed to the cuff by overmoulding in a subsequent step (see below).

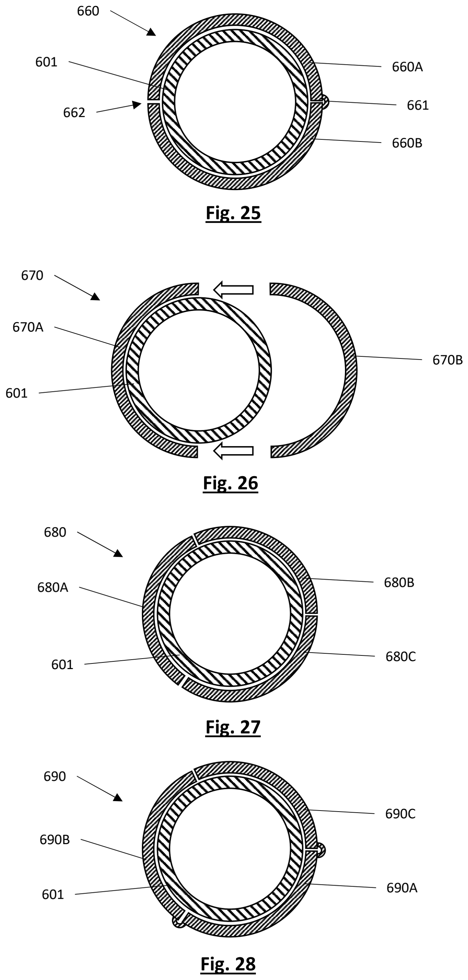

In embodiments according to the disclosure, the strain relief portion may comprise at least one such hinged shell. Figs. 25-28 show schematically some alternative embodiments, in cross-section.

In the embodiment of Fig. 25, the strain relief portion 660 is composed of one, first shell 660A which is integrally formed with the connector portion and hingedly connected thereto, and a second shell 660B which is integrally formed with the first shell and hingedly connected thereto. The first shell 660A may be like the ones 560A, 560B shown in Figs. 17-24, comprising a semi-cylindrical portion at one end of a leg with the hinge connection with the connector portion at the other end of the leg, so that the strain relief portion is spaced from the connector portion and a portion of the hose extremity 601 remains exposed for being fixed to the cuff by overmoulding. The second shell 660B may be a semi-cylindrical portion which is hingedly connected, by a hinge 661, to the first shell 660A at one side and provided with a snap connection mechanism 662 at the other side, by which the two shells are snap-fitted to each other. So in this embodiment, the connector portion (not shown), the first shell 660A and the second shell 660B are in this embodiment likewise integral parts which may be manufactured in a single injection moulding step.

In the embodiment of Fig. 26, the strain relief portion 670 is composed of one, first shell 670A which is integrally formed with the connector portion (not shown) and connected thereto, for example hingedly connected thereto, and a second shell 670B which is a separate part from the first shell 670A and connected thereto by means of a snap connection mechanism. The first shell 670A may be like the ones shown in Figs. 17-24, comprising a semi-cylindrical portion at one end of a leg, with the hinge connection with the connector portion at the other end of the leg, so that the strain relief portion is spaced from the connector portion and a portion of the hose extremity 601 remains exposed for being fixed to the cuff by overmoulding. In this embodiment, the hinge is not essential and the leg may be connected to the connector portion by a solid, non-pliable connection. The second shell 670B may be a separate semi-cylindrical portion with both shells 670A, 670B having a complementary snap connection mechanism in the same way as shown in Figs. 17-24, by which the two shells are snap-fitted to each other. So in this embodiment, the connector portion (not shown) and the first shell 670A are integral parts and the second shell 670B is a separate part. These parts may yet be manufactured in a single injection moulding step with the second shell for example connected to the other parts via a thin, separable connection.

The embodiment of Fig. 27 is an alternative to that of Figs. 17-24, wherein the strain relief portion 680 is composed of three shells 680A, 680B, 680C instead of two. Each shell may be similar to the ones shown in Figs. 17-18, comprising a 120° cylindrical portion at one end of a leg, with the hinge connection with the connector portion (not shown) at the other end of the leg. The three shells 680A, 680B, 680C may likewise be provided with complementary snap connection means, so that they can be snap fitted to each other for forming the strain relief portion.

The embodiment of Fig. 28 is an alternative to that of Fig. 25, comprising a first shell 690A which is integrally formed with, for example hingedly connected to, the connector portion (not shown) and two second shells 690B, 690C which are hingedly connected to the first shell. The two second shells 690B, 690C may likewise be provided with complementary snap connection means, so that they can be snap fitted to each other for forming the strain relief portion 690.

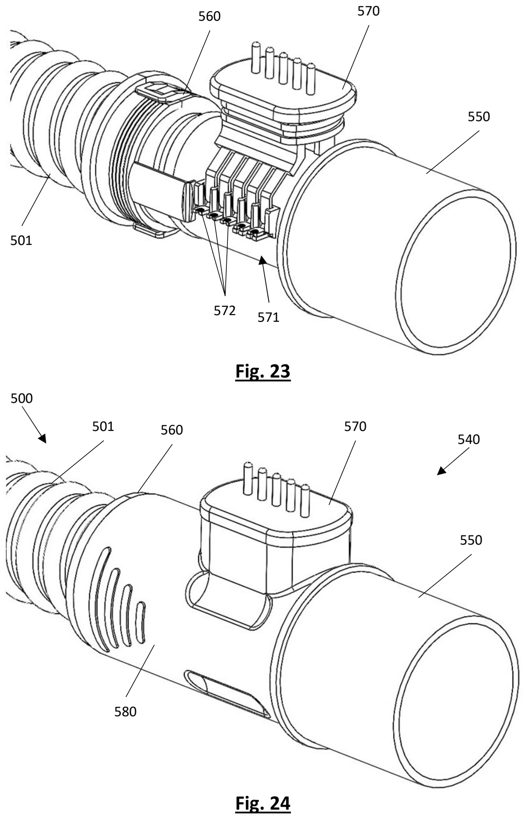

In each of these embodiments, the manufacturing process may comprise a further step wherein an overmoulded portion 580 is made, covering at least part of the connector portion 550 and/or at least part of the strain relief portion 560, 660. This overmoulding step also fixes the hose extremity 501, 601 to the cuff by bonding of the overmould material with the exposed portion of the hose extremity. In other words, the overmoulded portion 580 may comprise an inner portion which extends through the opening between the connector portion 550 and the strain relief portion 560 and is bonded to the tubular body 501, 601. The finished cuff is shown in Fig. 24.

### Connecting the wires/connectors to interface

With reference to Figs. 17-24 another aspect according to the present disclosure is described, which may be combined with other aspects and embodiments described herein, relating to steps for connecting the conductors 530 of the tubular body 501 to a cable interface 570 on the hose cuff. The steps are explained in respect of a flexible plastic hose as disclosed herein, but may generally be applied on any flexible plastic hose comprising a flexible hose wall comprising a thin-walled tubular body 101, 201, 401, 501 with a helical outer support rib and a plurality of conductors helically disposed within said support rib. The cuff 540 comprises a cable interface portion 570 provided for connecting a cable to the cuff and a platform portion 571 where the conductors 530 are connected to connector pads 572 of the cable interface. The platform portion comprises a rib section with a series of ribs 573 for guiding the individual conductors 530 and separating the conductors from each other, wherein each rib comprises a hook portion 574 at a distal end, each for holding one of the conductors 530.

The platform portion 571 may be an integral part of the connector portion 550 described herein above. As shown, the connector portion may comprise an insert portion 575 which is inserted into the hose extremity 501 and on which the platform portion 571 with the ridges 573 is provided. Preferably, at least one turn of the rib 520 is placed around the insert 571 portion. This may however be carried out in different ways.

The flexible hoses as disclosed herein, with the wires or conductors included in the grooves of the lower rib portion, have the advantage that the conductors are located in well-defined positions at the hose extremity, which was not the case in prior art hoses. These well-defined positioning of the wires or conductors may enable automation of the steps for connecting the wires or conductors to other components in the cuff, such as the conductive pads of the cable interface as explained herein.

As shown in Figs. 17-22, each of the conductors 530 of the hose is separated from the rib and guided along one of the ridges 573 of the platform portion 571 and underneath and around a hook portion 574 at the far end of the respective ridge. The cable interface portion 570 is placed adjacent to the platform portion with the connectors pads 572 adjacent to the hook portions 574. Each connector pad 572 of the cable interface portion comprises a slot for engaging one of the conductors 530. So each conductor 530 is provided along one of the ribs 573, behind the respective hook portion 574 and into a respective one of the slots of the connector pads 572. This has the advantage that a strong mechanical connection, or at least a well-defined position of the conductors 530 on the connector pads 572 may already be achieved.

As shown in Fig. 23, the conductors 530 are subsequently welded to the connector pads 572. In view of the well-defined positions, this step may also be automated and for example performed by means of ultrasonic welding. The excess wire is cut away after the welding step.

As shown in Fig. 24, in a final step an overmoulded portion 580 may be added to the cuff 540 by means of an injection moulding step. The overmould covers the exposed conductive parts (conductors and pads) to insulate and protect them from the environment. In the embodiment shown, the overmould also covers the strain relief portion 560 up to an annular ridge at the entrance side of the cuff, and an inner portion of the overmould 580 bonds with the hose extremity 501 and fixes the latter on the cuff, as described elsewhere herein.

### Experiments

The improvements in smoothness of the interior wall of the hoses as disclosed herein are illustrated by means of experiments.

The results are shown in the table 1 below. Sample 1 is a prior art hose which is commercially available, namely the Resmed Climateline Air Heated Tubing Airsense 10. Sample 2 is a hose manufactured according to the embodiment of Figs. 1-6, with the same diameter and length as Sample 1. The test results show a small improvement in smoothness (reduced flow resistance) in straight condition, with the improvement becoming more prominent when the flow increases and/or when the hose is bent. The test has been performed according to the standardized test procedure ISO5367_2014, §5.5.3 + Annex G: Test for increase in flow resistance with bending.

**Table 1**

| | Length (mm) | flow | Resistance to flow straight | | Resistance to flow with bending | |
|---|---|---|---|---|---|---|
| | | | Direction 1 | Direction 2 | Direction 1 | Direction 2 |
| | | l/min | hPA/l/min | hPA/l/min | % | % |
| Sample 1 | 1865 | 30 | 0.008 | 0.009 | 123 | 125 |
| Sample 2 | 1865 | 30 | 0.008 | 0.008 | 120 | 123 |

### Further embodiments

Embodiments of the present disclosure are formulated as follows:
Clause 1. A flexible plastic hose comprising a flexible hose wall formed of a helically wound ribbon and a helical outer support rib, and a plurality of conductors helically disposed within said support rib,
   wherein adjacent windings of the ribbon are attached to each other by means of a weld material such that an elongate tubular body is formed having a substantially smooth bore,
   wherein the support rib is positioned on top of the weld material and comprises a lower rib portion, comprising a plurality of grooves wherein the conductors are disposed, and an upper rib portion on top of the lower rib portion and the conductors, and
   wherein the weld material attaches the lower rib portion on the tubular body.
Clause 2. The flexible plastic hose according to clause 1, wherein the weld material forms a lower layer of the support rib, underneath the lower rib portion.
Clause 3. The flexible plastic hose according to clause 1 or 2, wherein the lower rib portion is a separate part from the helically wound ribbon, attached thereto by means of the weld material.
Clause 4. The flexible plastic hose according to clause 3, wherein the adjacent windings of the ribbon have opposite side edges which are spaced from each other in axial direction of the hose and are attached to each other by means of the weld material.
Clause 5. The flexible plastic hose according to clause 4, wherein a gap of a distance D is present between the opposite side edges of the adjacent windings of the ribbon, wherein the distance D is chosen in a range of 50% to 200%, preferably 75% to 150% of the thickness of the ribbon at the side edges.
Clause 6. The flexible plastic hose according to clause 5, wherein the weld material at least partly fills the gap between the opposite side edges of the adjacent windings of the ribbon, preferably substantially completely fills said gap.
Clause 7. The flexible plastic hose according to clause 1 or 2, wherein the lower rib portion is an integral part of the helically wound ribbon.
Clause 8. The flexible plastic hose according to clause 7, wherein the adjacent windings of the ribbon have opposite side edges which overlap each other in axial direction of the hose and are attached to each other by means of the weld material.
Clause 9. The flexible plastic hose according to clause 7 or 8, wherein a bottom side of the lower rib portion comprises a recess for accommodating an opposite side edge portion of the ribbon and the weld material.
Clause 10. The flexible plastic hose according to clause 7 or 8, wherein a bottom side of the lower rib portion is slanted and an opposite side edge portion of the ribbon is correspondingly tapered.
Clause 11. The flexible plastic hose according to any one of the preceding clauses, wherein the ribbon and the lower rib portion are made of a first thermoplastic material.
Clause 12. The flexible plastic hose according to any one of the preceding clauses, wherein the weld and the upper rib portion are made of a second thermoplastic material, preferably different from the first thermoplastic material.
Clause 13. A method of manufacturing a flexible plastic hose comprising a flexible hose wall formed of a helically wound ribbon and a helical outer support rib, and a plurality of conductors helically disposed within said support rib, the method comprising:
   helically winding the ribbon;
   attaching adjacent windings of the ribbon by means of a weld material such that an elongate tubular body is formed having a substantially smooth bore;
   positioning a lower rib portion of a support rib on top of the weld material, wherein the lower rib portion comprises a plurality of grooves;
   disposing the conductors in the grooves; and
   covering the lower rib portion and the conductors by means of an upper rib portion; wherein the weld material attaches the lower rib portion on the tubular body.
Clause 14. The method of clause 13, wherein the adjacent windings of the ribbon are not overlapped and wherein the weld material attaches the adjacent windings of the ribbon and the lower rib portion to each other.
Clause 15. The method of clause 13, wherein the lower rib portion is an integral part of the helically wound ribbon and wherein the adjacent windings of the ribbon are overlapped.
Clause 16. A flexible plastic hose according to any one of clauses 1-12, comprising a cuff connected to a first extremity of the tubular body and comprising: a connector portion through which the tubular body is connectable to an external device and a strain relief portion located at an entrance side of the cuff where the tubular body enters the cuff, the strain relief portion having, at least partially, an internal screw-thread complementary to the helical reinforcing rib and provided for holding a portion of the first extremity of the tubular body without being bonded thereto, wherein the strain relief portion comprises at least one shell which is integrally formed with the connector portion and hingedly connected thereto and which covers at least part of the tubular body in circumferential direction.
Clause 17. The flexible plastic hose of clause 16, wherein the strain relief portion comprises two or more of such shells which are hingedly connected to the connector portion, preferably wherein the shells together cover substantially the whole tubular body in circumferential direction, preferably wherein the shells are fixed to each other by means of one or more snap connectors.
Clause 18. The flexible plastic hose of clause 16, wherein the strain relief portion comprises at least one of said shells which is/are integrally formed with the connector portion and hingedly connected thereto, and at least one separate portion preferably fixed to the at least one shell by means of a snap connector, preferably wherein the shell and the separate portion together cover substantially the whole tubular body in circumferential direction.
Clause 19. The flexible plastic hose of clause 16, wherein the strain relief portion comprises a first shell which is hingedly connected to the connector portion and at least one second shell which is/are hingedly connected to the first shell, preferably wherein at least two of the first and second shells are fixed to each other by means of a snap connector, preferably wherein the first and second shells together cover substantially the whole tubular body in circumferential direction.
Clause 20. The flexible plastic hose of any one of clauses 16-19, wherein the cuff comprises an overmoulded portion which surrounds at least a portion of the connector portion and at least a portion of the strain relief portion, including the/each hinged connection.
Clause 21. A method of manufacturing a cuff onto a tubular body of a hose according to any one of the clauses 1 to 12, wherein the method comprises the steps of:
   providing a cuff part comprising a connector portion and a strain relief portion, wherein the strain relief portion has, at least partially, an internal screw-thread complementary to the helical support rib and provided for holding a portion of a first extremity of the tubular body without being bonded thereto, wherein the strain relief portion comprises at least one part which is integrally formed with the connector portion and hingedly connected thereto;
   mounting the connector portion onto the tubular body and subsequently moving the at least one part of the strain relief portion into position around the tubular body by hinging said at least one part around the respective hinge connection; and
   overmoulding the cuff part to obtain an overmoulded portion which surrounds at least a portion of the connector portion and at least a portion of the strain relief portion, including the/each hinged connection.
Clause 22. The flexible plastic hose of any one of clauses 1-12, wherein the hose comprises a cuff connected to a first extremity of the tubular body and comprising a cable interface portion provided for connecting a cable to the cuff and a platform portion where the conductors are connected to connector pads of the cable interface, wherein over a first portion at the first extremity of the tubular body the helical outer support rib is flattened with respect to a second, subsequent portion of the tubular body.
Clause 23. The flexible hose of clause 22, wherein the height of the helical outer support rib over the first portion of the tubular body is reduced by XXX of the height of the helical outer support rib over the second portion of the tubular body.
Clause 24. The flexible hose of clause 22 or 23, wherein the width of the helical outer support rib over the first portion of the tubular body is increased by XXX of the width of the helical outer support rib over the second portion of the tubular body.
Clause 25. A method of manufacturing a hose according to any one of the clauses 22-24, wherein the flattening of the helical outer support rib is achieved by pressing a roller element onto the outside of the rib while the rib material is in molten state.
Clause 26. The flexible plastic hose of any one of clauses 1-12, wherein the hose comprises a cuff connected to a first extremity of the tubular body and comprising: a cable interface portion provided for connecting a cable to the cuff and a platform portion where the conductors are connected to connector pads of the cable interface, wherein the platform portion comprises a rib section with a series of ribs for guiding the individual conductors and separating the conductors from each other, wherein each rib comprises a hook portion at a distal end for holding one of the conductors, and wherein each connector pad of the cable interface portion comprises a slot for engaging one of the conductors, and wherein each conductor extends along one of the ribs, behind the respective hook portion and into a respective one of the slots.
Clause 27. The flexible plastic hose of clause 26, wherein the cuff further comprises an overmoulded portion which covers exposed parts of the conductors and the connector pads.

## Claims

1. A flexible plastic hose comprising a flexible hose wall comprising a thin-walled tubular body with a helical outer support rib and a cuff connected to a first extremity of the tubular body, the cuff comprising a connector portion through which the tubular body is connectable to an external device and a strain relief portion located at an entrance side of the cuff where the tubular body enters the cuff, wherein the strain relief portion has, at least partially, an internal screw-thread complementary to the helical reinforcing rib and provided for holding a portion of the first extremity of the tubular body without being bonded thereto, **characterised in that** the strain relief portion comprises at least one shell which is integrally formed with the connector portion and is hingedly connected thereto and which covers at least part of the tubular body in circumferential direction.

2. The flexible plastic hose of claim 1, wherein the strain relief portion comprises two or more of such shells which are hingedly connected to the connector portion.

3. The flexible plastic hose of claim 2, wherein the shells together cover substantially the whole tubular body in circumferential direction

4. The flexible plastic hose of claim 2 or 3, wherein the shells are fixed to each other by means of one or more snap connectors.

5. The flexible plastic hose of claim 1, wherein the strain relief portion comprises at least one of said shells which is/are integrally formed with the connector portion and hingedly connected thereto, and at least one separate portion.

6. The flexible plastic hose of claim 5, wherein the separate portion is fixed to the at least one shell by means of a snap connector.

7. The flexible plastic hose of claim 5 or 6, wherein the shell and the separate portion together cover substantially the whole tubular body in circumferential direction.

8. The flexible plastic hose of claim 1, wherein the strain relief portion comprises a first shell which is hingedly connected to the connector portion and at least one second shell which is/are hingedly connected to the first shell.

9. The flexible plastic hose of claim 8, wherein at least two of the first and second shells are fixed to each other by means of a snap connector.

10. The flexible plastic hose of claim 8 or 9, wherein the first and second shells together cover substantially the whole tubular body in circumferential direction.

11. The flexible plastic hose of any one of claims 1-10, wherein the cuff comprises an overmoulded portion which surrounds at least a portion of the connector portion and at least a portion of the strain relief portion, including the/each hinged connection.

12. The flexible plastic hose of any one of claims 1-11, wherein the tubular body comprises a plurality of conductors helically disposed within said support rib.

13. The flexible plastic hose of claim 12, wherein the cuff comprises a cable interface portion provided for connecting a cable to the cuff and a platform portion where the conductors are connected to connector pads of the cable interface, wherein the platform portion comprises a rib section with a series of ribs for guiding the individual conductors and separating the conductors from each other, wherein each rib comprises a hook portion at a distal end for holding one of the conductors, and wherein each connector pad of the cable interface portion comprises a slot for engaging one of the conductors, and wherein each conductor extends along one of the ribs, behind the respective hook portion and into a respective one of the slots.

14. The flexible plastic hose of claim 13, wherein the cuff further comprises an overmoulded portion which covers exposed parts of the conductors and the connector pads.

15. A method of manufacturing a cuff onto a tubular body of a flexible hose, wherein the tubular body comprises a helical outer support rib, wherein the method comprises the steps of:
providing a cuff part comprising a connector portion and a strain relief portion, wherein the strain relief portion has, at least partially, an internal screw-thread complementary to the helical support rib of the tubular body and provided for holding a portion of a first extremity of the tubular body without being bonded thereto, wherein the strain relief portion comprises at least one part which is integrally formed with the connector portion and is hingedly connected thereto;
mounting the connector portion onto the tubular body and subsequently moving the at least one part of the strain relief portion into position around the tubular body by hinging said at least one part around the respective hinge connection; and
overmoulding the cuff part to obtain an overmoulded portion which surrounds at least a portion of the connector portion and at least a portion of the strain relief portion, including the/each hinged connection.
